# EUROPEAN PATENT APPLICATION

(11) **EP 3 777 828 A1**
(43) Date of publication of application: **17.02.2021**
(21) Application number: 19803576.8
(22) Date of filing: 17.05.2019
(51) Int. Cl.: A61K 9/00, A61K 9/127, A61K 45/06, A61P 35/00

(54) **LUNG SURFACTANT-BASED ANTICANCER DRUG**

(30) Priority: 18.05.2018 KR 20180057010
(71) Applicant: Korea Advanced Institute Of Science And Technology, Daejeon, 34141 (KR); Korea University Research and Business Foundation, Seoul 02841 (KR)
(72) Inventor: PARK, Ji Ho, Daejeon 34141 (KR); LIM, Jiyoung, Daejeon 34141 (KR); OH, Chan Hee, Daejeon 34141 (KR); JUNG, Daeho, Daejeon 34141 (KR); KIM, Hyun Koo, Seoul 02841 (KR); QUAN, Yu Hua, Seoul 02841 (KR)
(74) Representative: EP&C
(86) International application number: PCT/KR2019/005923
(87) International publication number: WO 2019/221544

(57) **Abstract**

The present invention relates to a pulmonary surfactant-based anticancer drug. The anticancer drug encapsulated in a liposome made of a pulmonary surfactant can effectively target lung cancer cells, especially adenocarcinomas derived from type II alveolar cells, and has low toxicity and excellent structural stability.

## Description

### BACKGROUND OF THE INVENTION

### 1. Field of the Invention

The present invention relates to a pulmonary surfactant-based anticancer drug.

### 2. Description of the Related Art

Cancer patients are increasing significantly every year, and lung cancer patients belong to the category with the highest mortality rate among all cancer patients.

Among lung cancers, adenocarcinoma accounts for about 40% of all lung cancers, and occurs mainly in the alveolar region. The origin of adenocarcinoma is often type II alveolar cells.

The type II alveolar cells secrete and store pulmonary surfactants, and the pulmonary surfactants control the tension of the lungs during the breathing process. The pulmonary surfactant is composed of lipid and membrane protein (Non-Patent Reference, EurRespir J. 1999 Jun; 13(6):1455-76).

When an anticancer drug is delivered to a target cell using such a pulmonary surfactant, it is expected that the anticancer drug can be effectively targeted to type II alveolar cells that secrete and store the pulmonary surfactant.

Therefore, the present invention is completed by preparing an anticancer drug encapsulated in a liposome made of a pulmonary surfactant.

### SUMMARY OF THE INVENTION

It is an object of the present invention to provide an anticancer drug that can be efficiently delivered to alveolar cells, has excellent biocompatibility, has low toxicity, and has excellent structural stability.

It is another object of the present invention to provide a method for preparing the anticancer drug.

To achieve the above objects, in an aspect of the present invention, the present invention provides a pharmaceutical composition for anticancer comprising a complex of a pulmonary surfactant derived from a living mammalian organ and an anticancer drug.

In another aspect of the present invention, the present invention provides a method for preparing the pharmaceutical composition for anticancer comprising the following steps:
preparing a first solution in which an anticancer drug is dissolved;
preparing a second solution in which a pulmonary surfactant is dissolved;
forming a film by mixing the first solution and the second solution, and drying the mixed solution; and
preparing a complex containing the anticancer drug by hydrating the film with distilled water.

### ADVANTAGEOUS EFFECT

The anticancer drug encapsulated in a liposome made of a pulmonary surfactant can effectively target lung cancer cells, especially adenocarcinomas derived from type II alveolar cells, and has low toxicity and excellent structural stability.

### BRIEF DESCRIPTION OF THE DRAWINGS

Figure 1 is a diagram illustrating a preparation process of a pulmonary surfactant-based particle encapsulated with a drug according to Example 1.
Figure 2 is a graph showing the results of confirming the amount of paclitaxel loaded in the particle after loading 1%, 2%, 5% or 10% (weight %) of paclitaxel relative to the mass of the particle prepared in Example 1, and removing the paclitaxel not loaded in the particle.
Figure 3 is a graph showing the results of confirming the amount of paclitaxel loaded in the particle after loading 1%, 2%, 5% or 10% (weight %) of paclitaxel relative to the mass of the particle prepared in Comparative Example 1, and removing the paclitaxel not loaded in the particle.
Figure 4 is a graph showing the results of evaluating the stability of the particles prepared in Example 1 and Comparative Example 1.
Figure 5 is a set of photographs showing the size and shape of the particles prepared in Example 1 and Comparative Example 1 taken through an electron microscope.
Figure 6 is a set of photographs showing the results of evaluation of intracellular uptake of the pulmonary surfactant particles labeled with a fluorescent dye.
Figure 7 is a set of photographs showing the results of comparing and evaluating the uptake efficiency between normal cells and cancer cells.
Figure 8 is a graph showing the results of confirming the toxicity of the particle itself.
Figure 9 is a set of photographs showing the results of evaluating the selectivity for A549 cell line among various lung cancer cell lines.
Figure 10 is a set of graphs showing the results of confirming the cytotoxicity specific to A549 cell line among various lung cancer cell lines.
Figure 11 is a graph showing the results of evaluating the DiR fluorescence signals observed over time after vaporizing the pulmonary surfactant particles of Example 1 labeled with DiR fluorescence and inhaling the particles into a mouse model.
Figure 12 is a diagram showing the preparation process of the A549 mouse lung cancer model and the experimental schedule.
Figure 13 is a set of photographs showing the results of an experiment to confirm the treatment efficacy in the A549 lung cancer model using the drug encapsulated particle.

### DESCRIPTION OF THE PREFERRED EMBODIMENTS

Hereinafter, the present invention is described in detail.

The present invention provides a pharmaceutical composition for anticancer comprising a complex of a pulmonary surfactant derived from a living mammalian organ and an anticancer drug.

At this time, the form of the complex is not particularly limited, but may be a liposome form, for example, or may be a form in which a pulmonary surfactant and an anticancer drug are bound through a covalent bond.

In addition, the pulmonary surfactant can be a lipoprotein complex produced in type II alveolar cells. That is, the pulmonary surfactant can include a mammalian pulmonary surfactant collected from the lung of a mammal. At this time, the mammal can be a human, and can be an animal other than a human, particularly a porcine or a bovine. In general, a natural pulmonary surfactant is secreted and stored in type II alveolar cells. Therefore, the pulmonary surfactant-based anticancer drug of the present invention can efficiently target alveolar cells to deliver the anticancer drug.

The term lipid in the lipoprotein complex means a natural, synthetic, or semi-synthetic (i.e., modified natural) compound that is generally amphiphilic. Lipid typically contains a hydrophilic component and a hydrophobic component. Exemplary lipids include phospholipids, fatty acids, fatty alcohols, triglycerides, phosphatides, oils, glycolipids, aliphatic alcohols, waxes, terpenes and steroids, but not always limited thereto. The phrase "semi-synthetic (or modified natural)" refers to a natural compound that has been chemically modified in some way.

Examples of phospholipids include natural and/or synthetic phospholipids.

Phospholipids that can be used herein include phosphatidyl choline (saturated and unsaturated), phosphatidyl glycerol, phosphatidyl ethanolamine, phosphatidyl serine, phosphatidic acid, phosphatidyl inositol, sphingolipids, diacyl glyceride, cardiolipin, ceramide and cerebroside, but not always limited thereto. Exemplary phospholipids include dipalmitoyl phosphatidyl choline (DPPC), dilauryl phosphatidyl choline (DLPC) (C12:0), dimyristoyl phosphatidyl choline (DMPC) (C14:0), distearoyl phosphatidyl choline (DSPC), dipitanoyl phosphatidyl choline, nonadecanoyl phosphatidyl choline, arachidoyl phosphatidyl choline, dioleoyl phosphatidyl choline (DOPC) (C18:1), dipalmitoleoyl phosphatidyl choline (C16:1), linoleoil phosphatidyl choline (C18:2), myristoyl palmitoyl phosphatidyl choline (MPPC), steroyl myristoyl phosphatidyl choline (SMPC), steroyl palmitoyl phosphatidyl choline (SPPC), palmitoyl oleoyl phosphatidyl choline (POPC), palmitoyl palmitoleoyl phosphatidyl choline (PPoPC), dipalmitoyl phosphatidyl ethanolamine (DPPE), palmitoyl oleoyl phosphatidyl ethanolamine (POPE), dioleoyl phosphatidyl ethanolamine (DOPE), dimyristoyl phosphatidyl ethanolamine (DMPE), distearoyl phosphatidyl ethanolamine (DSPE), dioleoyl phosphatidyl glycerol (DOPG), palmitoyl oleoyl phosphatidyl glycerol (POPG), dipalmitoyl phosphatidyl glycerol (DPPG), dimyristoyl phosphatidyl glycerol (DMPG), distearoyl phosphatidyl glycerol (DSPG), dimyristoyl phosphatidyl serine (DMPS), distearoyl phosphatidyl serine (DSPS), palmitoyl oleoyl phosphatidyl serine (POPS), soybean lecithin, egg yolk lecithin, sphingomyelin, phosphatidyl inositol, diphosphatidyl glycerol, phosphatidyl ethanolamine, phosphatidic acid and egg phosphatidyl choline (EPC), but not always limited thereto.

Examples of fatty acids and fatty alcohols include sterol, palmitic acid, cetyl alcohol, lauric acid, myristic acid, stearic acid, phytanic acid and dipalmitic acid, but not always limited thereto. Exemplary fatty acids include palmitic acid.

Examples of fatty acid esters include methyl palmitate, ethyl palmitate, isopropyl palmitate, cholesteryl palmitate, palmityl palmitate, sodium palmitate, potassium palmitate and tripalmitin, but not always limited thereto.

Meanwhile, the pulmonary surfactant contains a membrane protein, and the presence of this membrane protein enables selective and effective targeting of adenocarcinoma derived from type II alveolar cells. The membrane protein can include one or more natural surfactant polypeptides selected from the group consisting of SP-A, SP-B, SP-C and SP-D, a portion thereof, or a mixture thereof. Exemplary peptides can include at least about 5, 10, 15, 20, 25, 30, 35, 40, 45, or 50 amino acid fragments of a natural surfactant polypeptide. Exemplary SP-B polypeptides can include at least about 5, 10, 15, 20, 25, 30, 35, 40, 45 or 50 amino acid fragments of SP-B. The SP-B peptide can be an amino-terminal peptide or a carboxy-terminal peptide. An exemplary SP-B peptide can be a 25-amino acids amino terminal peptide.

In another embodiment, the pulmonary surfactant can include a recombinantly produced surfactant polypeptide. Recombinant SP-A, SPB, SP-C, SP-D or a portion thereof can be obtained by expressing a DNA sequence encoding SP-A, SP-B, SP-C, SP-D or a portion thereof in a suitable prokaryotic or eukaryotic expression system using various informed techniques. Recombinant vectors easily adapted to contain an isolated nucleic acid sequence encoding a surfactant polypeptide or a portion thereof, host cells containing the recombinant vectors, and methods of preparing such vectors and host cells, and their use in the production of encoded polypeptides by recombinant techniques are well known to those in the art. A nucleic acid sequence encoding a surfactant polypeptide or a portion thereof can be provided in an expression vector comprising a nucleotide sequence encoding a surfactant polypeptide operably linked to at least one regulatory sequence. It should be understood that the design of the expression vector may depend on factors such as the selection of host cells to be transformed and/or the type of protein to be expressed. The vector copy number, the ability to control the copy number, and the expression of any other protein encoded by the vector (e.g., antibiotic marker) should be considered. For example, in order to produce a protein or a polypeptide including a fusion protein or a polypeptide, the nucleic acid can be used to induce the expression and overexpression of the kinase and phosphatase polypeptides in cells grown in culture.

In order to express a surfactant polypeptide or a portion thereof, host cells can be transfected with a recombinant gene. The host cell can be any prokaryotic or eukaryotic cell. For example, the polypeptide can be expressed in bacterial cells such as E. coli, insect cells, yeasts or mammalian cells. In these examples, if the host cell is a human cell, it may or may not be in a living subject. Other suitable host cells are known to those in the art. In addition, the host cells can be supplemented with tRNA molecules that are not typically found in the host, in order to optimize the expression of the polypeptide. Other methods suitable for maximizing the expression of the polypeptide are known to those of in the art.

Methods of producing polypeptides are well known in the art. For example, the host cells transfected with an expression vector encoding a surfactant polypeptide or a portion thereof can be cultured under the suitable conditions to allow the expression of the polypeptide. The polypeptide can be secreted and isolated from a medium mixture of containing cells and the polypeptide. Alternatively, the polypeptide can remain cytoplasmically intact. Then, the cells are collected, lysed, and the protein isolated from the cell lysate.

The surfactant polypeptide and the surfactant lipid interact by hydrostatic interaction. Charged amino acids interact with the polar head groups of lipids, and hydrophobic amino acids interact with phospholipid acyl side chains. For example, SP-B and SP-C are hydrophobic proteins. Both SP-B and SP-C bind preferentially to anionic lipids (e.g., phosphatidylglycerol (PG), not DPPC). SP-A and SP-D are hydrophilic proteins and interact with a wide range of amphiphilic lipids including glycerophospholipids, sphingophospholipids, sphingoglycolipids, lipid A and lipoglycans. SP-A binds to DPPC. For example, a hydrostatic interaction of KL4, an SP-B mimetic, and lipids in a natural surfactant or lipids contained in a surface active agent is observed. The lysine residue in the KL4 peptide interacts with the charged head group of DPPC, and the hydrophobic leucine residue interacts with the phospholipid acyl side chain of phosphatidylglycerol.

Meanwhile, the anticancer drug can be encapsulated in an amount of 0.1 to 10 weight parts per 100 weight parts of a liposome made of a pulmonary surfactant, but not always limited thereto. The loading amount of the anticancer drug can be appropriately adjusted according to the patient's condition. In addition, the diameter of the complex can be 200 to 400 nm, 220 to 400 nm, 240 to 400 nm, 260 to 400 nm, 280 to 400 nm, 300 to 400 nm, 320 to 400 nm, 200 to 380 nm, 200 to 360 nm, 200 to 340 nm, 200 to 320 nm, 220 to 380 nm, 240 to 360 nm, 260 to 340 nm, and 280 to 320 nm, but not always limited thereto.

The anticancer drug can be used without limitation, as long as it is an informed anticancer drug. At this time, specific examples of the anticancer drug include a hydrophobic anticancer drug, a cancer immunotherapy, and the like.

Specific examples of the hydrophobic anticancer drug are as follows: paclitaxel, doxorubicin, cis-platin, docetaxel, tamoxifen, camtothecin, anasterozole, carboplatin, topotecan, belotecan, irinotecan, gleevec and vincristine.

In one aspect, the anticancer drug can be delivered to the lungs by inhalation. Inhalation devices, such as inhalers (including dry powder inhalers and metered dose inhalers) and nebulizers (also known as atomizers) can be used to deliver the anticancer drug to the lung.

An exemplary dry powder inhaler can be obtained from Inhale Therapeutic Systems. The dry powder inhaler can also be obtained from 3M.

In another aspect of the present invention, the present invention provides a method for preparing the pharmaceutical composition for anticancer comprising the following steps:
preparing a first solution in which an anticancer drug is dissolved;
preparing a second solution in which a pulmonary surfactant is dissolved;
forming a film by mixing the first solution and the second solution, and drying the mixed solution; and
preparing a complex containing the anticancer drug by hydrating the film with distilled water.

At this time, the hydration can be performed at a temperature of 40 to 90°C, and more preferably at a temperature of 60 to 70°C.

In addition, after performing all of the above steps, a step of adjusting the diameter of the anticancer drug can be further performed, and an extruder kit can be used for the purpose of adjusting the diameter.

The anticancer drug encapsulated in a liposome made of a pulmonary surfactant can effectively target lung cancer cells, especially adenocarcinomas derived from type II alveolar cells, and has low toxicity and excellent structural stability. These effects are demonstrated by the examples and experimental examples described later.

Hereinafter, the present invention will be described in detail by the following examples and experimental examples.

However, the following examples and experimental examples are only for illustrating the present invention, and the contents of the present invention are not limited thereto.

### Example 1: Preparation of pulmonary surfactant-based particles encapsulated with a drug

Pulmonary surfactant-based particles encapsulated with a drug were prepared by the following process using paclitaxel, a water insoluble drug having strong anticancer efficacy and very high hydrophobicity, as the drug.

Paclitaxel powder was dissolved in methanol at a concentration of 10 mg/ml (first solution).

Pulmonary surfactant powder (manufacturer: Mitsubishi / product name: Surfacten) was dissolved in a solution of chloroform and methanol in a volume ratio of 2:1 (v:v) at a concentration of 10 mg/ml (second solution).

The first solution and the second solution were mixed so that the pulmonary surfactant and paclitaxel were mixed in a mass ratio of 20:1 (w:w), and the mixed solution was put in a glass bottle and dried. At this time, if necessary, the mass ratio of mixing the pulmonary surfactant and paclitaxel can be adjusted within an appropriate range for optimal drug loading.

The dried pulmonary surfactant was hydrated with distilled water to a concentration of 3 mg/ml. The hydration was carried out on a hot plate and the temperature was maintained at 60-70°C. The particles produced during the hydration process were made to have an average diameter of 400 nm using an extruder kit.

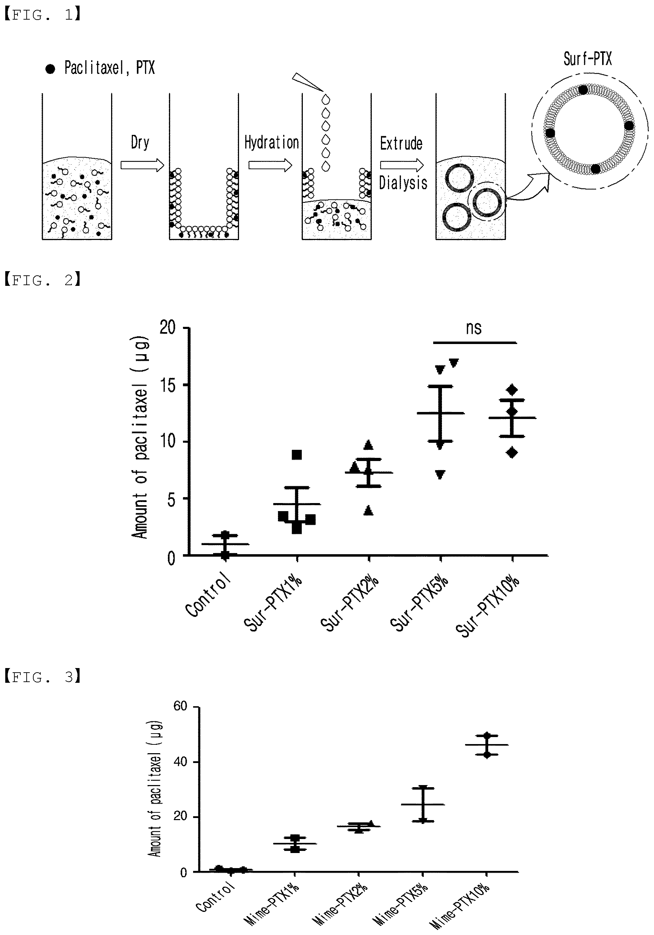

Thereafter, the drug that could not be encapsulated in the particles was separated for 12 hours through dialysis using a 100 kDa membrane. As a result, the pulmonary surfactant particles encapsulated with a drug (paclitaxel) were prepared (Figure 1).

In the case of preparing the pulmonary surfactant-based particles encapsulated with the drug by mixing the pulmonary surfactant and the drug in a mass ratio of 20:1 as in Example 1, the drug in the final prepared particles can be encapsulated in about 1 weight% by the mass of the particles themselves. That is, in Example 1, the drug was encapsulated at 1 weight part based on 100 weight parts of the particle itself.

On the other hand, in order to observe the intracellular uptake described later, pulmonary surfactant-based particles encapsulated with a dye were prepared by the following process using DiI, a water insoluble dye having very high hydrophobicity, as the dye.

The red dye DiI was dissolved in methanol at a concentration of 1 mg/ml (first solution).

Pulmonary surfactant powder was dissolved in a solution of chloroform and methanol in a volume ratio of 2:1 at a concentration of 10 mg/ml (second solution).

The first solution and the second solution were mixed so that the pulmonary surfactant and dye were mixed in a mass ratio of 1000:1, and the mixed solution was put in a glass bottle and dried.

The dried pulmonary surfactant was hydrated with distilled water to a concentration of 3 mg/ml. The hydration was carried out on a hot plate and the temperature was maintained at 60-70°C. The particles produced during the hydration process were made to have an average diameter of 400 nm using an extruder kit.

### Example 2: Preparation of pulmonary surfactant-based particles not encapsulated with a drug

Pulmonary surfactant particles not encapsulated with a drug were prepared by performing the same process as described in Example 1 except that paclitaxel was not used.

### Comparative Example 1: Preparation of pulmonary surfactant-based mimetic particles encapsulated with a drug

In order to compare the function of the 'pulmonary surfactant-based particles encapsulated with a drug' prepared in Example 1, mimetic particles were artificially prepared through the following process.

Particularly, mimetic particles were prepared using only DPPC (lipid), DOPC (lipid), DPPG (lipid) and cholesterol, which are the main components of the pulmonary surfactant. The main difference between the pulmonary surfactant-based particles according to Example 1 and the mimetic particles according to Comparative Example 1 was the presence or absence of a membrane protein.

DPPC, DOPC, DPPG and cholesterol were mixed at a molar ratio of 52.2:22.1:11.2:14.4, and the mixture was dissolved in chloroform (third solution).

The first solution of Example 1 and the third solution prepared above were mixed so that the mimetic particle components (DPPC, DOPC, DPPG and cholesterol) and the drug were mixed in a mass ratio of 20:1, and the mixed solution was put in a glass bottle and dried.

The mimetic particle components were hydrated with distilled water to a concentration of 3 mg/ml. The hydration was carried out on a hot plate and the temperature was maintained at 60-70°C. The particles produced during the hydration process were made to have an average diameter of 400 nm using an extruder kit.

Thereafter, the drug that could not be encapsulated in the particles was separated for 12 hours through dialysis using a 100 kDa membrane. As a result, the pulmonary surfactant mimetic particles encapsulated with a drug (paclitaxel) were prepared.

On the other hand, in order to observe the intracellular uptake described later, mimetic particles encapsulated with DiI were prepared in the same manner as described in Example 1.

### Comparative Example 2: Preparation of pulmonary surfactant-based mimetic particles not encapsulated with a drug

Pulmonary surfactant mimetic particles not encapsulated with a drug were prepared by performing the same process as described in Comparative Example 1 except that paclitaxel was not used.

### Experimental Example 1: Evaluation of particle size and surface charge

The size and surface charge of the particles prepared in Examples 1 and 2 and Comparative Examples 1 and 2 were measured using DLS (Dynamic Light Scattering). The results are shown in Table 1.

**[Table 1]**

| | Example 1 (5 wt% of PTX loaded) | Example 2 (PTX not loaded) | Comparative Example 1 (5 wt% of PTX loaded) | Comparative Example 2 (PTX not loaded) |
|---|---|---|---|---|
| Mean diameter (nm) | 424.9 | 321.8 | 322.6 | 301.3 |
| Surface charge, ZP (mV) | -53.5 | -45.6 | -36.8 | -40 |

### Experimental Example 2: Evaluation of the concentration of a drug encapsulated in particles

The concentration of the drug encapsulated in the pulmonary surfactant particles prepared in Example 1 was quantified and evaluated by HPLC. The results are shown in Figure 2.

Figure 2 is a graph showing the results of confirming the amount of paclitaxel loaded in the particle after loading 1%, 2%, 5% or 10% (weight %) of paclitaxel relative to the mass of the particle prepared in Example 1, and removing the paclitaxel not loaded in the particle.

As shown in Figure 2, when more than 5 weight% was used, the amount loaded was saturated, although there was a slight variation.

The drug was loaded with about 12 µg per 1 mg of the pulmonary surfactant.

Therefore, it was judged that it was most efficient to load the drug at a concentration of 5 weight%, and the experiment was conducted in this way in the future.

Meanwhile, the concentration of the drug encapsulated in the particles prepared in Comparative Example 1 was also quantified and evaluated, and the results are shown in Figure 3.

Figure 3 is a graph showing the results of confirming the amount of paclitaxel loaded in the particle after loading 1%, 2%, 5% or 10% (weight %) of paclitaxel relative to the mass of the particle prepared in Comparative Example 1, and removing the paclitaxel not loaded in the particle.

As shown in Figure 3, unlike the pulmonary surfactant, a lot of drugs were loaded even at a concentration of 5% or more. This was because the mimetic particles did not have membrane proteins that the conventional pulmonary surfactant had, so there was a lot of space for drugs to be loaded.

In the case of the mimic particles, about 25 µg of the drug was loaded per 1 mg of the pulmonary surfactant.

### Experimental Example 3: Evaluation of particle stability and confirmation by electron microscopy

The particles prepared in Example 1 and Comparative Example 1 were mixed each with FBS (Fetal Bovine Serum) (10% of the total volume). Thereafter, each of them was placed in a 37°C incubator, and the size of the particles for each time period was measured using DLS equipment.

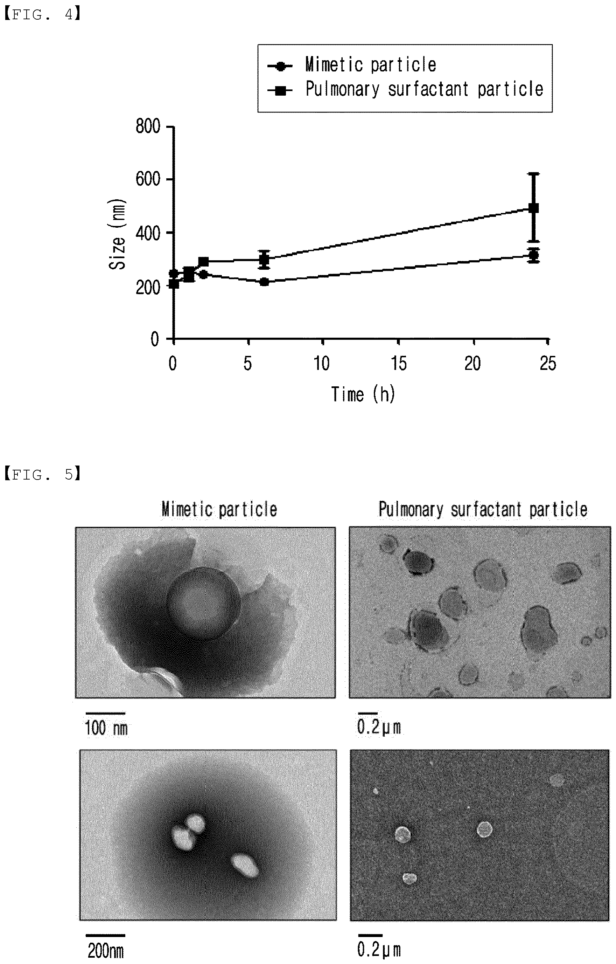

Figure 4 is a graph showing the results of evaluating the stability of the particles prepared in Example 1 and Comparative Example 1.

As shown in Figure 4, the particles prepared in Example 1 and Comparative Example 1 had very little change in particle size occurring over time. Therefore, it was confirmed that the particle stability was excellent.

Thereafter, for electron microscopy, the particles and glutaraldehyde were mixed in a volume ratio of 2.5% and fixed at room temperature for 2 hours. Then, the particles were put on a carbon grid and stained with a 2% PTA solution. The results are shown in Figure 5.

Figure 5 is a set of photographs showing the size and shape of the particles prepared in Example 1 and Comparative Example 1 taken through an electron microscope.

As shown in Figure 5, it was confirmed that the particles prepared in Example 1 and Comparative Example 1 were circular particles having a size of 200 to 300 nm.

### Experimental Example 4: Evaluation of intracellular uptake of pulmonary surfactant particles

Since the pulmonary surfactant is derived from alveolar type 2 cells, the present inventors tried to prove that the surfactant is well ingested in A549 cells, the same type of alveolar type 2 cells.

### - A549: human lung adenocarcinoma cell line from type 2 alveolar cell

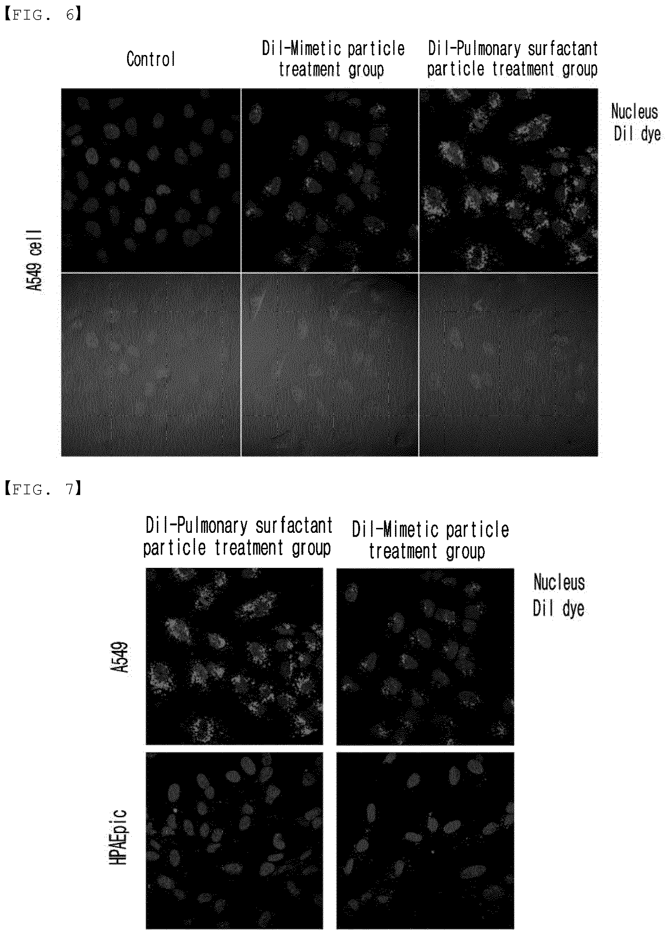

A549 cells were subcultured in a 6 well plate at the density of 20,000 cells/well, and one day later, the particles of Example 1 or Comparative Example 1 (3 mg/ml) containing DiI (red fluorescence) were treated thereto at a concentration of 60 µg/ml. The plate was placed in a 37°C incubator for 4 hours, and then the medium was replaced. Cell nuclei were stained with Hoechst dye 24 hours after the medium was replaced. The degree of intracellular uptake was confirmed using a confocal microscope (60x lens). The results are shown in Figure 6.

Figure 6 is a set of photographs showing the results of evaluation of intracellular uptake of the pulmonary surfactant particles loaded with a drug.

As shown in Figure 6, in the case of the mimetic particles of Comparative Example 1, since the affinity with A549 cells was low, the intracellular uptake was not made much.

On the other hand, in the case of the pulmonary surfactant-based particle of Example 1 treated with the same concentration, it was confirmed that the relatively much more intracellular uptake was made compared to that of Comparative Example 1.

This is because the membrane protein in the pulmonary surfactant, which is a specific property of the pulmonary surfactant, played an important role in the intracellular uptake.

### Experimental Example 5: Comparative evaluation of uptake efficiency between normal cells and cancer cells

To evaluate whether the pulmonary surfactant-based particles encapsulated with the drug prepared in Example 1 and the pulmonary surfactant-based mimetic particles encapsulated with the drug prepared in Comparative Example 1 had a difference in uptake efficiency between normal cells and cancer cells, the following experiment was performed.

HPAEpic (Human Pulmonary Alveolar Epithelial Cells, normal cells) or A549 cells used in the above experimental examples were seeded in a 6-well plate at the density of 20,000 cells/well, and the DiI-labeled liposome particles of Example 1 and Comparative Example 1 (3 mg/ml) were added thereto at a concentration of 60 µg/ml. After 4 hours, the medium was washed, and after 24 hours, the results were observed under a confocal microscope. The results are shown in Figure 7.

Figure 7 is a set of photographs showing the results of comparing and evaluating the uptake efficiency between normal cells and cancer cells.

As shown in Figure 7, it was confirmed that most of the particles of Example 1 and Comparative Example 1 did not remain in the normal cells HPAEpic (Human Pulmonary Alveolar Epithelial Cells), and it was also confirmed that the particles of Example 1 and Comparative Example 1 were accumulated in relatively large amounts in the lung cancer cells A549. It is understood that the above results are due to the fact that normal cells have an active function of absorbing and discharging the particles, whereas cancer cells have the property of continuously retaining the particles after absorbing them.

When comparing the particles of Example 1 and Comparative Example 1, it was confirmed that a relatively large amount of the particles of Example 1 were accumulated in cancer cells compared to the mimetic particles of Comparative Example 1.

### Experimental Example 6: Particle toxicity test

In order to evaluate the toxicity of the particles and drugs in the particles, the following experiment was performed.

A549 cells were subcultured in a 96 well plate at the density of 3,000 cells/well, and a day later, the pulmonary surfactant-based particles and mimetic particles were treated thereto at various concentrations.

First, in order to confirm the toxicity of the particles themselves, the pulmonary surfactant-based particles of Example 2 and the mimetic particles of Comparative Example 2 without a drug were treated to the cells at the following concentrations (Conc: 1 µg/ml, 5 µg/ml, 10 µg/ml, 25 µg/ml, 50 µg/ml, 75 µg/ml, 150 µg/ml, 300 µg/ml, 600 µg/ml, and 1200 µg/ml).

The plate was placed in a 27°C incubator for 4 hours, and then the medium was replaced. Twenty four hours after the medium was replaced, the degree of cell death was confirmed by MTT assay. The results are shown in Figure 6.

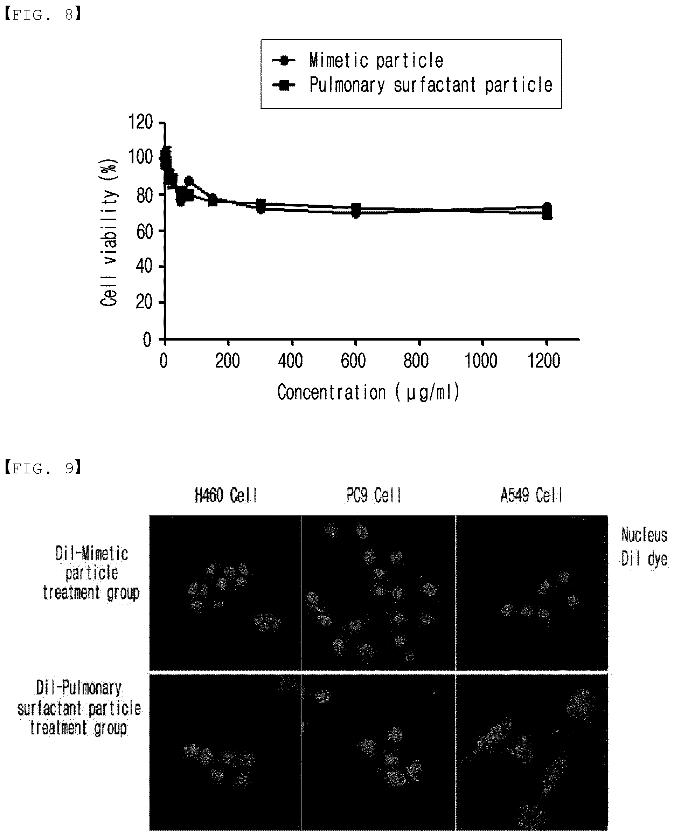

As shown in Figure 8, it was confirmed that more than 70% of the cells were survived up to 1200 µg/ml of both particles.

### Experimental Example 7: Evaluation of selectivity for A549 cell line among various lung cancer cell lines

The following experiment was performed to confirm whether the pulmonary surfactant-based particles encapsulated with a drug prepared in Example 1 and the drug prepared in Comparative Example 1 exhibit specific selectivity to A549 cell line among various lung cancer cell lines.

The following cell lines were seeded in a 6-well plate at the density of 5,000 cells/well, to which the DiI-labeled liposome particles of Example 1 and Comparative Example 1 were treated at a concentration of 100 µg/ml. After 2 hours, the medium was washed, and the cell nuclei were stained with Hoechst dye, followed by observation under a confocal microscope. The results are shown in Figure 9.
- A549 cell line (Adenocarcinoma / Human lung cancer)
- H460 cell line (Large cell carcinoma / Human lung pleural effusion)
- PC9 cell line (Adenocarcinoma / Human lung cancer)

Figure 9 is a set of photographs showing the results of evaluating the selectivity for A549 cell line among various lung cancer cell lines.

As shown in Figure 9, it was confirmed that the particles according to Example 1 were significantly absorbed in A549 cell line than H460 and PC9 lung cancer cell lines.

In addition, the following experiment was performed to confirm whether the pulmonary surfactant-based particles encapsulated with a drug prepared in Example 1 and the drug prepared in Comparative Example 1 exhibit specific selectivity to A549 cell line among various lung cancer cell lines.

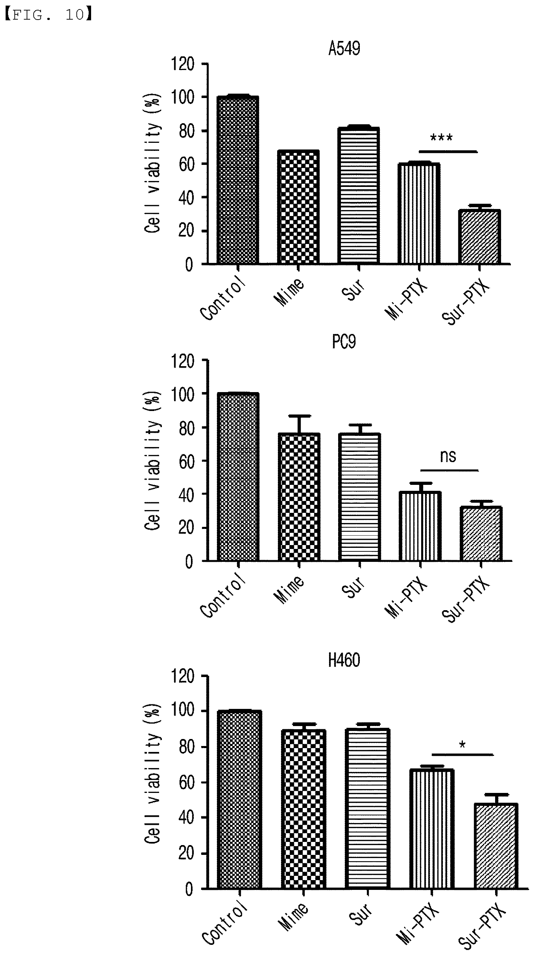

The lung cancer cell lines were seeded in a 96-well plate at the density of 3,000 cells/well, to which the liposome particles of Example 1 and Comparative Example 1 were treated at a concentration of 15 µg/ml. After 2 hours, the medium was washed and the cell viability (%) was evaluated. The results are shown in Figure 10.

Figure 10 is a set of graphs showing the results of confirming the cytotoxicity specific to A549 cell line among various lung cancer cell lines. (Mime and Sur are the particles of Comparative Example 2 and Example 2, respectively; and Mi-PTX and Sur-PTX are the particles of Comparative Example 1 and Example 1, respectively.)

As shown in Figure 10, it was confirmed that the drug-encapsulated pulmonary surfactant-based particles prepared in Example 1 showed remarkable cytotoxicity to A549 cell line than H460 or PC9 lung cancer cell line. The above results are due to the fact that the pulmonary surfactant particles are more ingested in A549 cells than the mimetic particles.

### Experimental Example 8: In vivo experiment using drug-encapsulated pulmonary surfactant

### <8-1> Particle stability test before/after vaporization

In the case of the *in vivo* experiment, an inhalation technique was used to deliver the particles to the lung of a mouse model. At this time, inExpose, SCIREQ (nebulizer) was used.

The device vaporizes liquid particles through sonication. At this time, the particle size was checked before/after the vaporization to ensure that the particles maintain the nano-sized shape. The vaporized particles were collected in a 50 ml tube and devolatized, and the size was measured with a DLS device. The results are shown in Table 2.

**[Table 2]**

| | Size before sonication (nm) | Size after sonication (nm) |
|---|---|---|
| Example 1 (Surfactant-PTX) | 223.4 | 185.8 |
| Example 2 (Surfactant) | 214.1 | 218.5 |
| Comparative Example 1 (Mimetic-PTX) | 216.1 | 188.3 |
| Comparative Example 2 (Mimetic) | 223.3 | 223 |

As shown in Table 2, even though the particles were vaporized, the particle size before/after the vaporization was almost unchanged. From the above results, it was confirmed that the nanoparticles according to the present invention were vaporized and stably delivered into the mouse model.

### <8-2> Confirmation of the residual degree of the vaporized particles in the lung of a mouse model

To confirm that the vaporized surfactant particles remained in the lung of the mouse model for a long time and were hardly transmitted to other organs, the surfactant was labeled with DiR fluorescence, and the particles were delivered to the mouse model through vaporization.

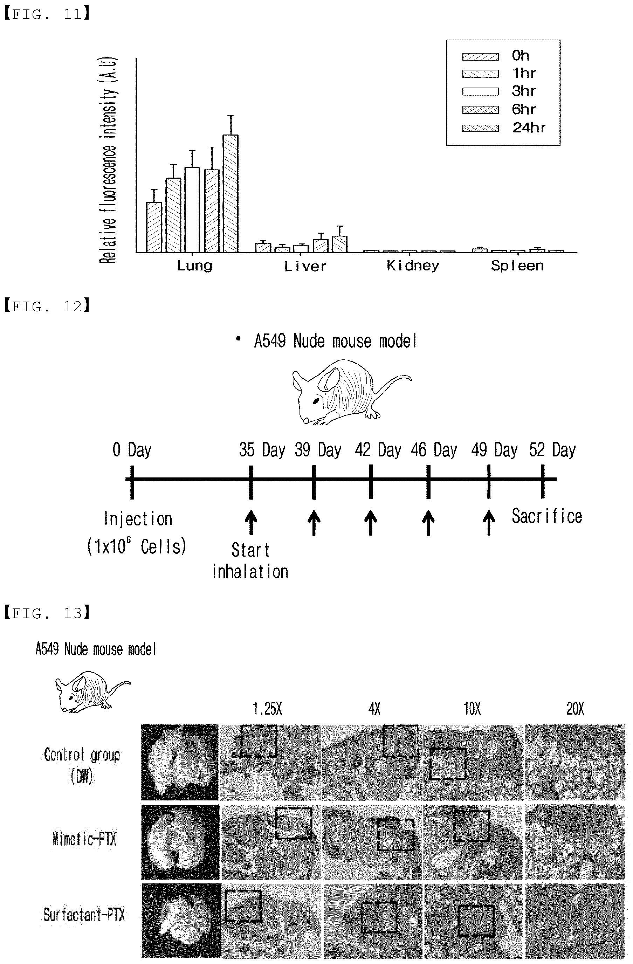

Organs were extracted from the mouse model immediately after the vaporization (0 h), after 1 hour, after 3 hours, after 6 hours, and after 24 hours, and then the DiR fluorescence signal (800 nm) was measured using a Li-CoR device. The results are shown in Figure 11.

Figure 11 is a graph showing the results of evaluating the DiR fluorescence signals observed over time after vaporizing the pulmonary surfactant particles of Example 1 labeled with DiR fluorescence and inhaling the particles into a mouse model.

As shown in Figure 11, it was confirmed that the vaporized particles remained in the lung for at least 24 hours and were hardly transmitted to other organs.

### <8-3> Confirmation of therapeutic efficacy in A549 lung cancer model using the drug-encapsulated particles

In order to construct an A549 lung cancer model, one million A549 cells were injected into the tail of a nude mouse by intravenous injection. About 5 weeks later, the anticancer drug was delivered at a concentration of 1 mg/kg twice a week using an inhalation technique. At this time, it was divided into the control group injected with water (DW), the group injected with the drug-encapsulated mimetic particles (Comparative Example 1, Mi-PTX), and the group injected with the drug-encapsulated pulmonary surfactant particles (Example 1, Sur-PTX). After inhaling about 5 times, the mouse model was sacrificed to extract the lung, and the lung was stained with H&E. Then, the therapeutic effect was confirmed through analysis by a pathologist.

The preparation process of the A549 mouse lung cancer model and the experimental schedule are shown in Figure 12, and the results of confirming the therapeutic effect are shown in Figure 13.

When the lung was analyzed by H&E staining, the dense circular area in the lung was the A549 lung cancer site. As shown in Figure 13, in the case of the control group (DW) and the group inhaling the drug-encapsulated mimetic particles (Comparative Example 1), many lung cancers in the lung were observed throughout the lung, and the therapeutic effect was insufficient. According to the opinion of the pathologist who analyzed the photographs, it was confirmed that when the drug-encapsulated pulmonary surfactant particles (Example 1) were inhaled, there was less lung cancers by 30% compared to the control group. Through this, it was confirmed that the drug-encapsulated pulmonary surfactant particles were effective in the treatment of the A549 lung cancer model.

### INDUSTRIAL APPLICABILITY

The complex in which an anticancer drug is encapsulated in a liposome made of a pulmonary surfactant can effectively target lung cancer cells, especially adenocarcinomas derived from type II alveolar cells, and has low toxicity and excellent structural stability, so that the complex can be effectively used as an anticancer composition.

## Claims

1. A pharmaceutical composition for anticancer comprising a complex of a pulmonary surfactant derived from a living mammalian organ and an anticancer drug.

2. The pharmaceutical composition for anticancer according to claim 1, wherein the pulmonary surfactant is a lipoprotein complex produced in type II alveolar cells.

3. The pharmaceutical composition for anticancer according to claim 1, wherein the pulmonary surfactant includes a membrane protein.

4. The pharmaceutical composition for anticancer according to claim 1, wherein the complex is **characterized by** targeting adenocarcinomas derived from type II alveolar cells.

5. The pharmaceutical composition for anticancer according to claim 1, wherein the complex is a complex in which the anticancer drug is encapsulated in the liposome made of the pulmonary surfactant derived from the living mammalian organ, and the anticancer drug is encapsulated in an amount of 0.1 to 10 weight parts based on 100 weight parts of the liposome made of the pulmonary surfactant.

6. The pharmaceutical composition for anticancer according to claim 1, wherein the complex has an average diameter range of 200 to 400 nm.

7. A method for preparing the pharmaceutical composition for anticancer of claim 1 comprising the following steps:
preparing a first solution in which an anticancer drug is dissolved;
preparing a second solution in which a pulmonary surfactant is dissolved;
forming a film by mixing the first solution and the second solution, and drying the mixed solution; and
preparing a complex encapsulated with the anticancer drug by hydrating the film with distilled water.

8. The method for preparing the pharmaceutical composition according to claim 7, wherein the hydration is performed at a temperature of 40 to 90°C.

9. The method for preparing the pharmaceutical composition according to claim 7, wherein the method further includes a step of adjusting the diameter of the complex after performing the step of hydrating.

10. The method for preparing the pharmaceutical composition according to claim 7, wherein the pulmonary surfactant includes a membrane protein.
